# EUROPEAN PATENT SPECIFICATION

(11) **EP 2 401 515 B1**
(45) Date of publication and mention of the grant of the patent: **02.12.2015**
(21) Application number: 10746796.1
(22) Date of filing: 24.02.2010
(51) Int. Cl.: F16B 35/04, F16B 35/00, F16C 1/00, A61B 17/86, F16B 5/02

(54) **FLEXIBLE SCREW**
FLEXIBLE SCHRAUBE
VIS FLEXIBLE

(30) Priority: 24.02.2009 US 155146 P
(43) Date of publication of application: 04.01.2012
(73) Proprietor: Flex Technology, Inc., Charlottesville, VA 22903 (US)
(72) Inventor: KRAUSE, William, R., Charlottesville VA 22902 (US); FARD, Mike, Charlottesville VA 22911 (US)
(74) Representative: Verhees, Godefridus Josephus Maria
(86) International application number: PCT/US2010/025284
(87) International publication number: WO 2010/099239

(56) References cited:
- EP-A2- 1 767 160
- WO-A1-2006/105935
- WO-A2-2007/117571
- US-A- 4 959 064
- US-A- 5 019 079
- US-A- 6 053 922
- US-A1- 2001 018 588
- US-A1- 2003 158 556
- US-A1- 2005 113 919
- US-A1- 2005 154 390

## Description

### Field of Invention

This invention relates to flexible screws; that is, those fastening devices by which can hold two pieces together.

### Description of the Related Art

A screw, or bolt, is a type of fastener characterized by a helical ridge, known as an external thread or just thread, wrapped around a cylinder. Some screw threads are designed to mate with a complementary thread, known as an internal thread, often in the form of a nut or an object that has the internal thread formed into it. Other screw threads are designed to cut a helical groove in a softer material as the screw is inserted. The most common uses of screws are to hold objects together and to position objects. Threaded fasteners either have a tapered shank or a non-tapered shank. Fasteners with tapered shanks are designed to either be driven into a substrate directly or into a pilot hole in a substrate. Mating threads are formed in the substrate as these fasteners are driven in. Fasteners with a non-tapered shank are designed to mate with a nut or to be driven into a tapped hole.

Flexible screws are screws in which the cylindrical portion of the device is bendable about the longitudinal length. Flexible screws are useable in many applications to join curved members together, to join misaligned holes, to absorb vibration between two components and numerous other applications. Although usable in many forms of bone connection, the bone screws disclosed are particularly useful in the intramedullary fixation of fractured or severed bone fragments

Bone screws are typically used in internal fixation to anchor the fixation system to the relevant bone portions or to join two or more fragments of a fractured bone into close proximity for bone union. For example, screws can be used in plate or rod systems to treat complex fractures of long bones or conditions such as vertebral instability. In small bone fractures, such as the bones of the hands, feet and wrist, the screw is placed across the fracture site to bring the fracture surfaces in close proximity.

In almost all bone connections of the kind being addressed in the pending application, it is essential that the fractured surfaces to be united are brought into close contact. This intimacy of contact is usually referred to as "compression". The actual need is for the fractured surfaces to be in close, well-fitting contact and to be so held during the healing process. In practice, the simplest way of ensuring this close contact is, where practical, to apply a compressive loading to the bone portions in a direction substantially normal to the fracture faces.

Regardless of the end use, the design of the screw is critical since the design will have a direct impact on the short term and long term viability of the screw as a means for anchoring fixation systems to bone. In terms of design, the screw is broken up into two major sections, a head segment which links to the fixation element, and a stem segment (or shaft) which anchors into the bone. The design of the shaft is particularly important in terms of short term and long term viability, with the short term stability dictated solely by mechanical considerations and the long term stability determined by a combination of mechanical (e.g. fatigue strength of the screw) and biological (e.g. bone/screw interface) considerations.

Hitherto, bone screws have been one of four typical forms. One of these has a thread only at its leading end, the head at the trailing end being separated from the thread by a smooth, cylindrical shank. It will be clear that such a bone screw, by threading wholly in the remote bone fragment and extending freely through the near fragment, can provide compressive action upon the fractured faces to be united.

The second type of bone screw has a cylindrical stem or shaft threaded over its full length. Such a screw can only be used to apply compression between two bone fragments if the near fragment is "over drilled" so that the thread engages solely in the remote fragment, the near fragment being free to move over the stem of the screw during insertion.

The third type of bone screw, commonly called a compression screw or Herbert screw (U.S. Pat. No. 4,175,555), has a region of large pitch and small diameter thread near the leading end and a region of smaller pitch and larger diameter thread near the trailing end, with the regions being separated by an unthreaded section. The Herbert screw, however, suffers from a number of disadvantages. In the Herbert screw, the leading threads have a smaller diameter than the trailing threads. This is necessary to permit the leading threads to pass through the relatively large bore in the near bone fragment and engage the smaller bore in the remote bone fragment. The larger trailing threads then engage the larger bore in the near bone fragment As a result of this arrangement, any stripping of the threads cut into the bones during installation of the screw occurs in the remote bone, causing the necessity of drilling another bore. When stripping occurs in the bore in the near bone fragment, a screw having a head thereon could still be used to compress the fracture even though the near bore was stripped. However, when stripping occurs in the bore in the remote bone, the option of using a standard screw with the head thereon is eliminated.

The fourth type of bone screw has a cylindrical or tapered stem or shaft threaded with a variable pitch, course at the leading end and decreasing toward the trailing end, over its entire length. The Huebner screw (U.S. Pat. No. 5,871 ,486), sold under the trademark ACUTRAK, in most versions, is fully threaded and has a changing pitch over the entire length. The outside diameter of the thread tapers from front to rear so that as the trailing threads ream the tracks left by the leading threads due to the pitch change, the trailing threads are expanding outward into undisturbed material. The ACUTRAK screw can be driven in as far as desired without reduction in compression because of the expanding thread diameter along its length. In addition, the screw generates compression over the entire length, rather than only at the tip and tail as with Herbert.

Although the ACUTRAK screw was a major improvement over the Herbert screw, installation of the ACUTRAK screw requires careful attention. In particular, the screw is typically installed in a tapered hole that has been pre-dhlled. Drilling this tapered hole can be difficult because the tapered drill bit sometimes clogs with bone and tapered bits require more pressure to feed than a similar straight drill. Moreover, for best results, the depth of the hole should generally match the length of the screw, requiring the surgeon to drill the hole accurately. The other area of concern during installation is the torque needed to drive the screw. When the screw reaches the point where the root and hole taper match, the driving force increases substantially. Similarly, in dense bone, the driving force may make installation difficult.

A common characteristic of all the screws described and other screws commercially available is that the shaft connecting the leading end to the trailing end is a straight, rigid structure. However the bone the screw is inserted into is usually a curved structure. Thus unless the screw is initially inserted precisely along the center axis of the bone after reducing the fracture, the screw will cause the bone to rotate to align itself with the screw thus causing the fracture to open. Another disadvantage of the prior art screws is that they generally follow the straight path and exit out of the side of the bone or enter the cortex of the bone, thus further weakening the bone. In US 2005/113919 A1 a spinal mobility preservation apparatus is disclosed. This known apparatus is a spacer designed to keep two components apart and allow movement between the two vertebras. WO2007/117571 discloses a compression screw according to the preamble of claim 1.

### SUMMARY OF THE INVENTION

The disclosed screw as specified in claim 1 provides a screw that is flexible and will follow the natural curvature of a bone to prevent unwanted penetration or increased erosion of the cortex, thereby overcoming the complications associated with a straight rigid screw inserted into a curved bone. The disclosed screw uses a modification of the flexible shaft technology as taught by Krause et al in US Patents 6,053,922 and 6,447,518 by imparting a serpentine, helical slot along a segment or segments of the component (screw) to form a flexible shaft. Preferably, the flexible shaft is formed by laser cutting an elongated tubular member of substantial wall thickness, to form the slot around and along the tubular member. A serpentine path can also be superimposed on a circumferential slot about the circumference of the shaft in the form of a generally sinusoidal wave. The sinusoidal wave forms dovetail-like teeth, which have a narrow base region and an anterior region that is wider than the base region. Thus, adjacent teeth interlock. The teeth can have a configuration as illustrated in U.S. Patent No. 4,328,839.

The present invention overcomes the deficiencies and problems evident in the prior art as described herein above by combining the following features into an integral, longitudinally, laterally and torsionally flexible segment of the component.

A slot of substantial length and width extends in a generally helical, serpentine or other predetermined path, either continuously or intermittently, around and along the tubular member. The slot can follow the pitch of the adjacent threads or be of a different pitch such that the slot cuts through the thread. Alternatively, the slot or series of slots can extend in a circumferential manner around the tubular member. Advantageously, the slot is cut at an angle normal to the shaft using a computer controlled cutting technique such as laser cutting, water jet cutting, milling or other means. Additionally, this slot may be cut at an angle to the normal so as to provide an undercut slot; preferably the angle is in the range from about 5 to about 45 degrees from the normal.

A plurality of slots can be employed thereby increasing the flexibility of the component, relative to a shaft having a single slot of identical pattern. The serpentine path forms a plurality of teeth and complimentary recesses on opposite sides of the slot. The slot has sufficient width to form an unbound joint permitting limited movement in any direction between the teeth and the recesses, thereby providing limited flexibility in all directions upon application of tensile, compressive, and/or torsion forces to said component. In a similar manner the slot can have increased width in one direction compared to another direction thus providing increased flexibility in one direction.

The flexible segment can have different degrees of flexibility along the length of said shaft. The varied flexibility can be achieved by having the pitch of the helical slot vary along the length of the shaft. The varied flexibility corresponds to the variation in the pitch of the helical slot. The helical path can have a helix angle in the range of about 10 degrees to about 45 degrees, and the helix angle can be varied along the length of the shaft to produce correspondingly varied flexibility. Alternatively, the width of the helical slot can vary along the length of the shaft to provide the varied flexibility. The rigidity of the flexible shaft can be achieved through the design of the slot pattern, thereby enabling the use of thinner walls than would otherwise be require to produce equivalent rigidity. In a preferred embodiment, the ratio of the amplitude of the serpentine path to the pitch of the slot is in the range from greater than 0.1 to about 0.8.

In one embodiment the slot can be filled with a resilient material, partially or entirely along the path of the slot. The resilient material can be an elastomer compound which can be of sufficient thickness to fill the slot and to encapsulate the entire shaft thus forming an elastomer enclosed member. The elastomer can be a resilient material such as a urethane or a silicone compound. The rigidity of the flexible shaft can be further achieved or varied through the use of filler material having different stiffness properties, thereby enabling the use of thinner walls than would otherwise be require to produce equivalent rigidity. The use of an elastomer is disclosed in US2008221620 and US8353935.

Preferably, the flexible segment is formed by laser cutting an elongated tubular member of substantial wall thickness, to form the slot around and along the tubular member in a helical manner. A serpentine path can be superimposed on a helical wave in the form of a generally sinusoidal wave. The slot may have the same pitch as the threads of the screw and be formed on the root diameter of the screw.

Preferably, the sinusoidal wave forms dovetail-like teeth, which have a narrow base region and an anterior region which is wider than the base region. Thus, adjacent teeth interlock. The teeth can have a configuration as illustrated in U.S. Patent No. 4328839.

An important aspect of this invention therefore lies in providing a screw for insertion in a curved bone that follows the curvature of the bone.

The flexible screw is a rigid material with a body having a diameter, a length, and multiple segments, at least one of which has at least one helical slot to form a flexible segment with exterior threads on at least one of the segments. The trailing segment has a receiving area to receive a rotational force device. The at least one helical slot can have a serpentine configuration with about 1 to about 10 cycles per longitudinal revolution. The body can be completely or partially hollow to receive elastomehc material that can fill at least a portion of the hollow body, fill at least a portion of the at least one slot, and/or encompass at least a portion of the body..

The segments that are the leading segment and trailing segment can each or both have at least one cutting recess. The leading segment and trailing segment can both have threads having the same or different pitch and amplitude than the threads of the other segment. Additionally, the second of at least one helical slot can a different pattern than, and be spaced from, the first of one helical slot in both the helical and serpentine patterns.

The body can be tapered or each segment can be of a different diameter than the other segments. Example combinations of flexible and nonflexible include:
1 ) a first, leading, segment having exterior threads, a second segments having a helical slot, a third trailing segment having exterior threads; 2) a first, leading, segment having a helical slot and exterior threads, a second segments having a helical slot, a third trailing segment having exterior threads; 3) a first, leading, segment having exterior threads, a second segments having a helical slot and exterior threads, a third trailing segment having exterior threads; 4) a first, leading, segment having exterior threads with a first diameter and a helical slot, a second segments having a helical slot and exterior threads having a second diameter, a third trailing segment having exterior threads and a third diameter; 5) a first, leading, segment having exterior threads and a helical slot, a second segments having a helical slot, a third trailing segment; 6) a first, leading, segment having exterior threads and a helical slot, a second segment and a third trailing segment.

### BRIEF DESCRIPTION OF THE DRAWINGS

Examples of the invention are illustrated in the drawings herewith. All of the figures are drawn on an oversized scale, and like structure in different figures bears like reference numerals.
Figure 1 is the compression bone screw as described by Herbert in US 4,175,555;
Figure 2 is the bone screw as described by Heubner in US Patent 5,871 ,486;
Figure 3 is an isometric view the first embodiment of the flexible bone screw as describe in the present application;
Figure 4 is a side elevation view of a flexible compression bone screw;
Figure 5 is a sectional view of the flexible compression bone screw through the longitudinal plane B-B from Figure 4;
Figure 6 is an exploded view of the section D shown in Figure 4;
Figure 7 is an isometric view the second embodiment of the flexible bone screw as describe in the present application;
Figure 8 is a side elevation view of a flexible compression bone screw shown in Figure 7;
Figure 9 is a sectional view of the flexible compression bone screw through the longitudinal plane B-B from Figure 7;
Figure 10 is the isometric view of the third embodiment of the flexible compression screw 100 having a tapered profile;

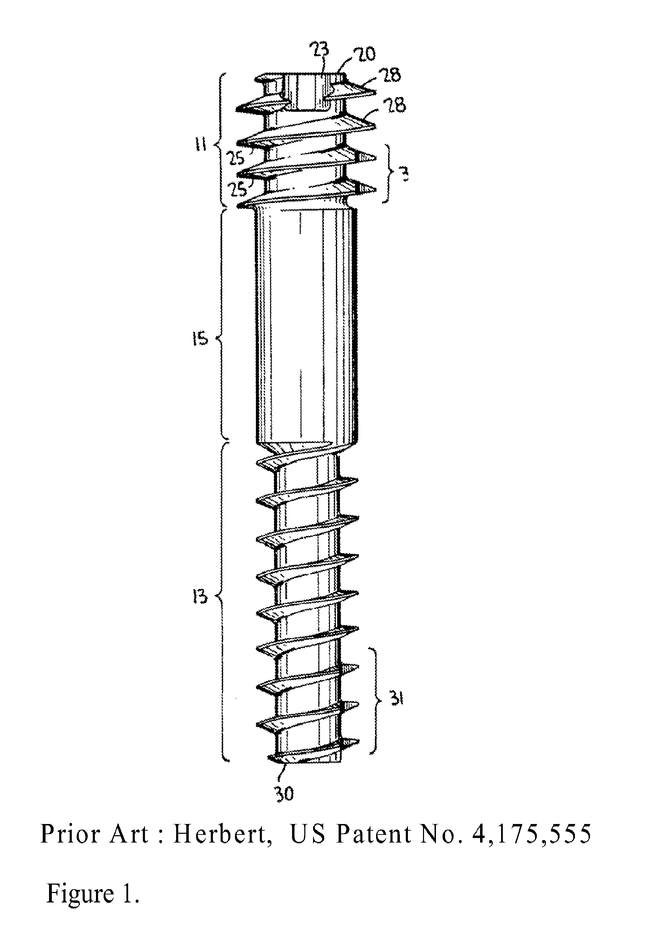

Figure 1 1 is side elevation of the tapered compression screw 100;

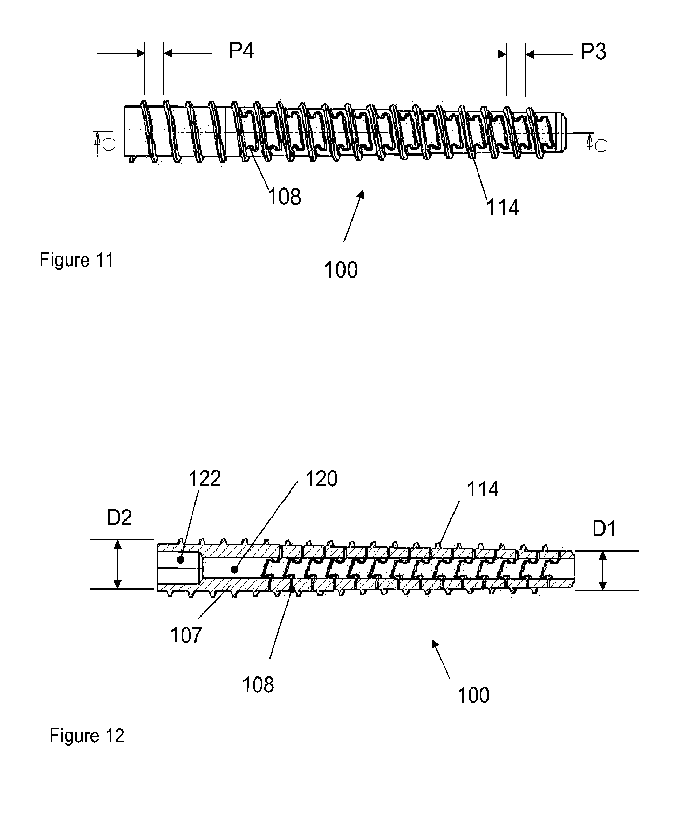

Figure 12 is a sectional view of the tapered compression screw 100 through the longitudinal plane C-C of Figure 1 1 ;

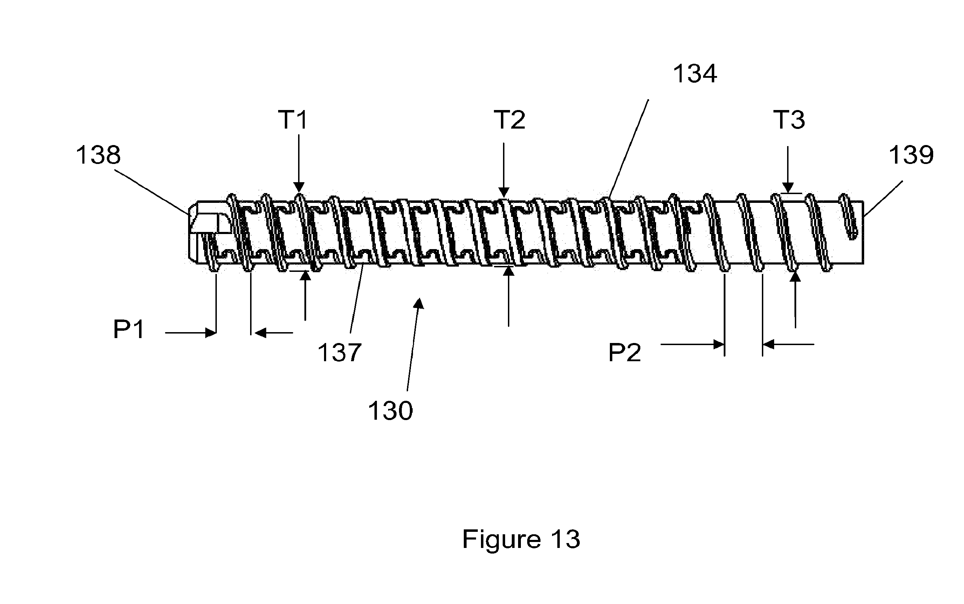

Figure 13 is a side elevation of a variable radius thread compression screw;

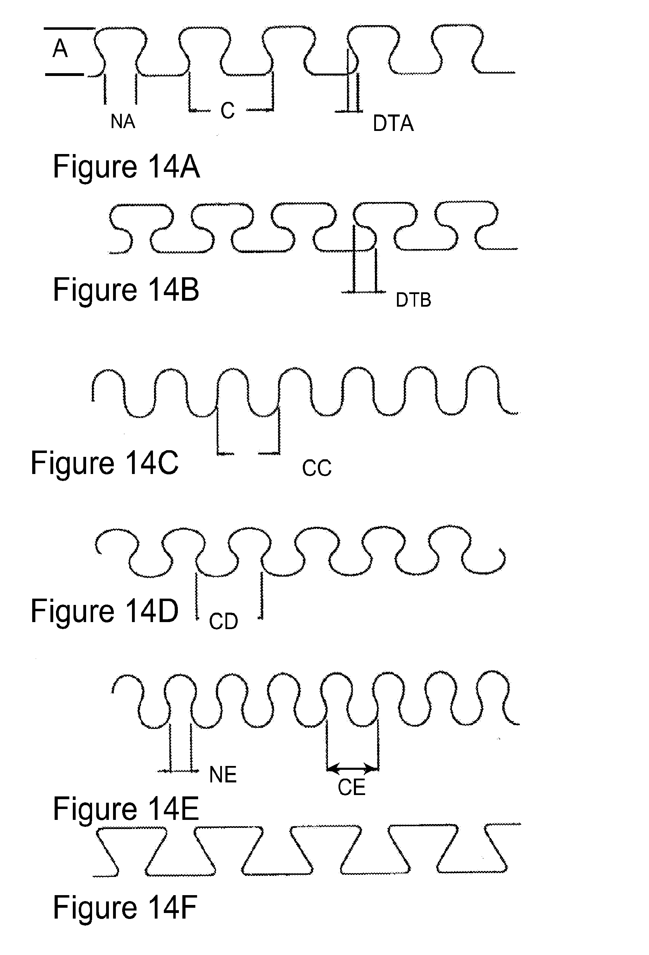

Figure 14a - 14k show schematic representations of additional spiral slot patterns in accordance with the invention;

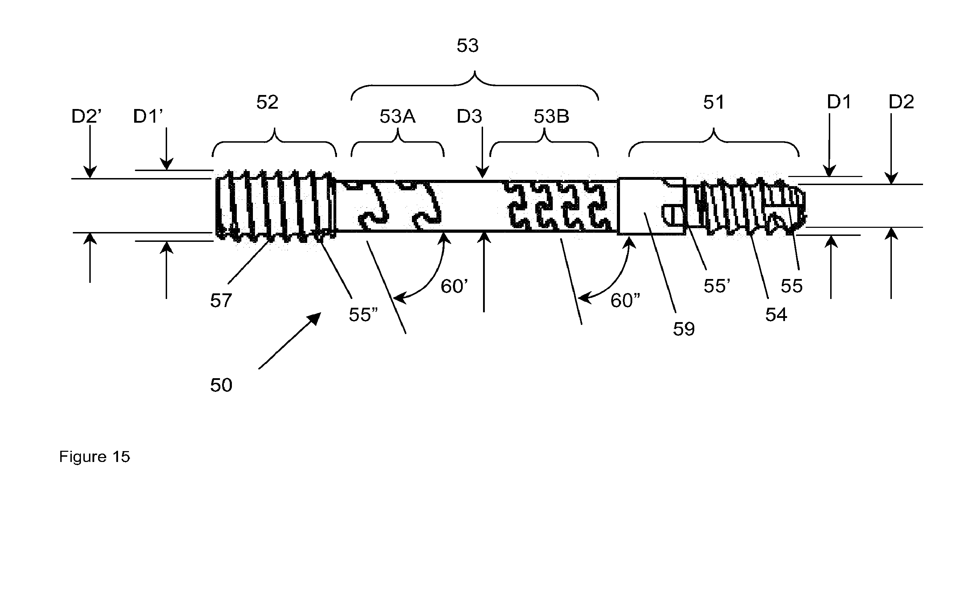

Figure 15 is a side view of another embodiment of the flexible screw illustrating dual serpentine patterns having separated by a nonflexible segment in accordance with the invention;

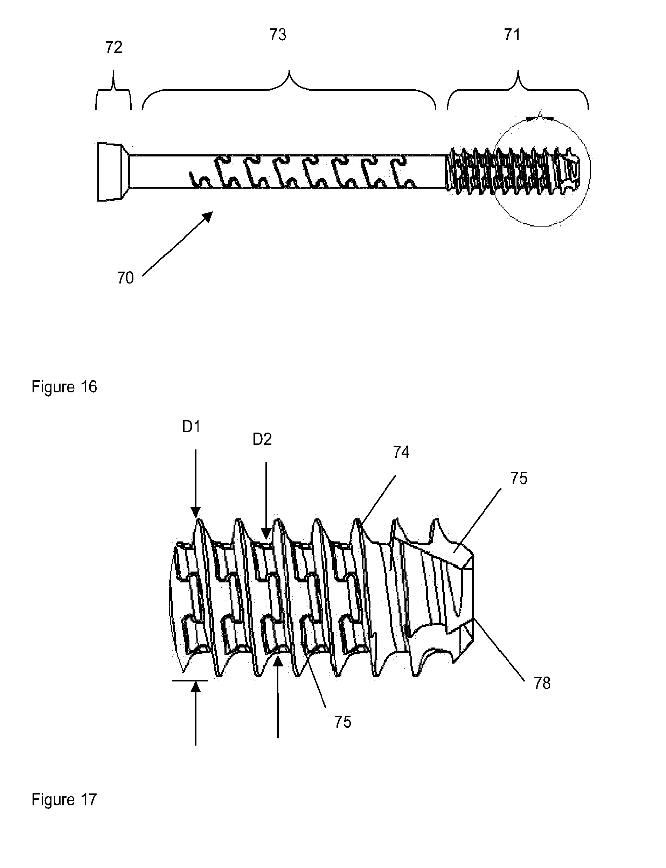

Figure 16 is a side view off an alternate embodiment having a threaded leading edge having a slot that, in accordance with the invention;
Figure 17 is a view of the threaded portion of the threaded leading edge of Fig. 16;

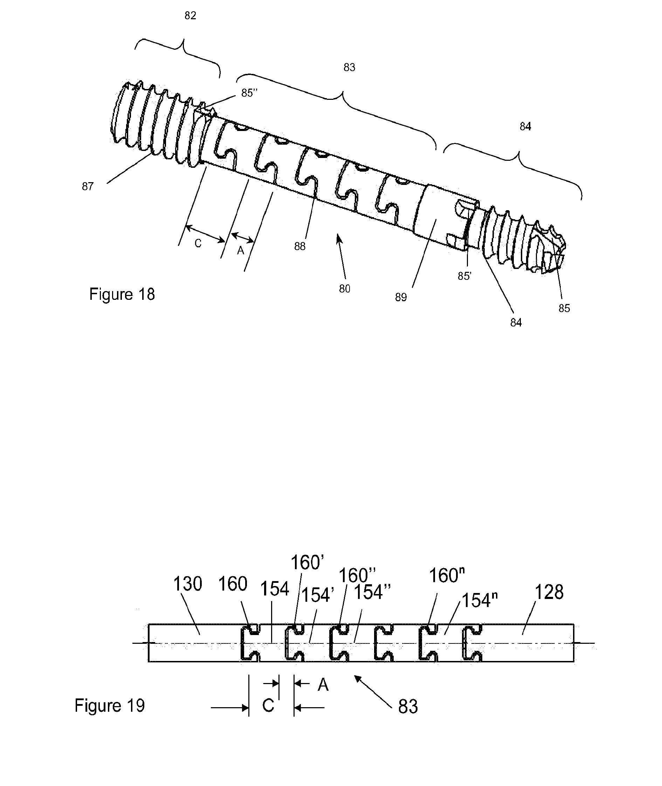

Figure 18 is a side view of an additional embodiment of the flexible screw in accordance with the invention;
Figure 19 is a schematic representation of the center segment of Figure 18, showing general pattern of the circumferential serpentine slots along the length of the rod in accordance with the invention;

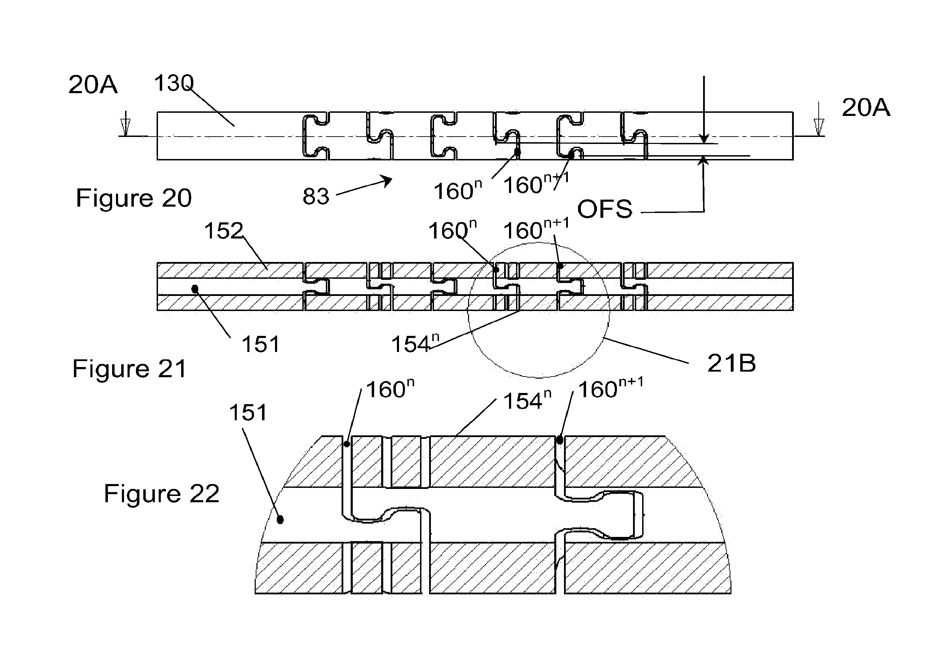

Figure 20 is an illustration of variation of the change in orientation of the serpentine slot relative to the adjacent slot whereby the teeth of each adjacent circumferential slot is staggered or offset a variable distance;
Figure 21 is a cross sectional view of the central segment through the longitudinal axis of Figure 20, showing general pattern of the offset serpentine, circumferential slots along the length of the segment in accordance with the invention;
Figure 22 is an exploded view of section 21 showing the gap and interlocking of the serpentine slot of two slots that have been offset or staggered;

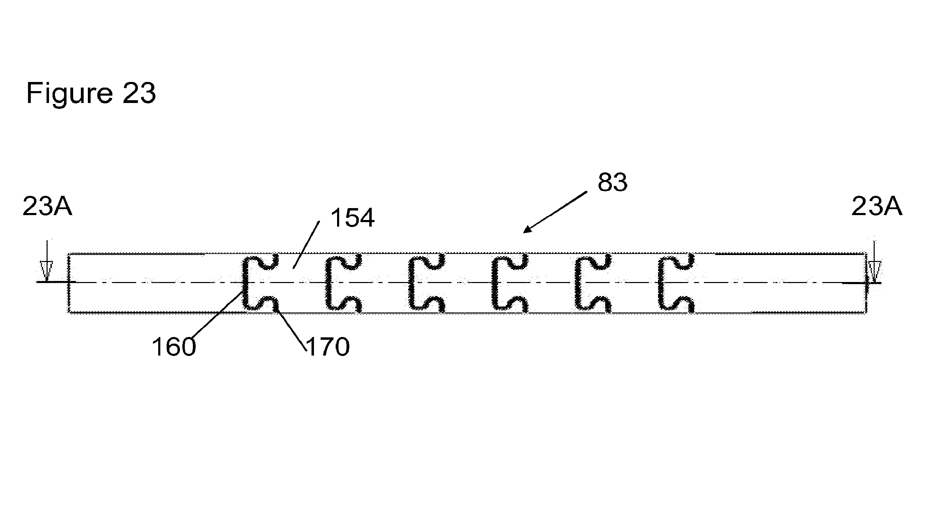

Figure 23 is a schematic representation of the central segment of Figure 18, showing general pattern of the circumferential serpentine slots with an elastomer filler material in the slot;

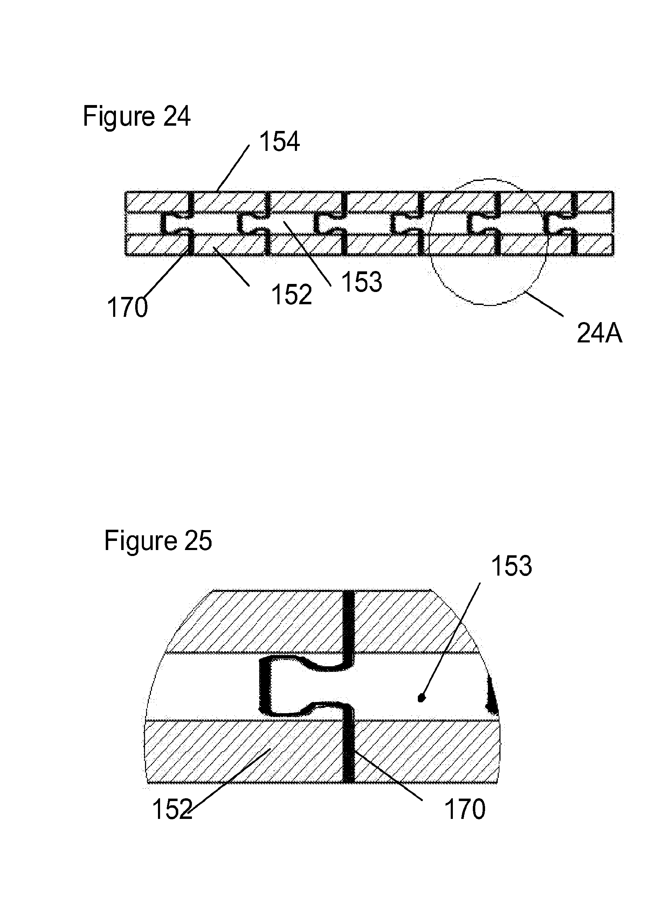

Figure 24 is a sectional illustration though the longitudinal axis 23A-23A shown in Figure 23 of the central segment showing the slot with a resilient filler in a portion of the slot in accordance with the invention;
Figure 25 is a magnified view of the area 24A in Figure 24 in accordance with the invention;

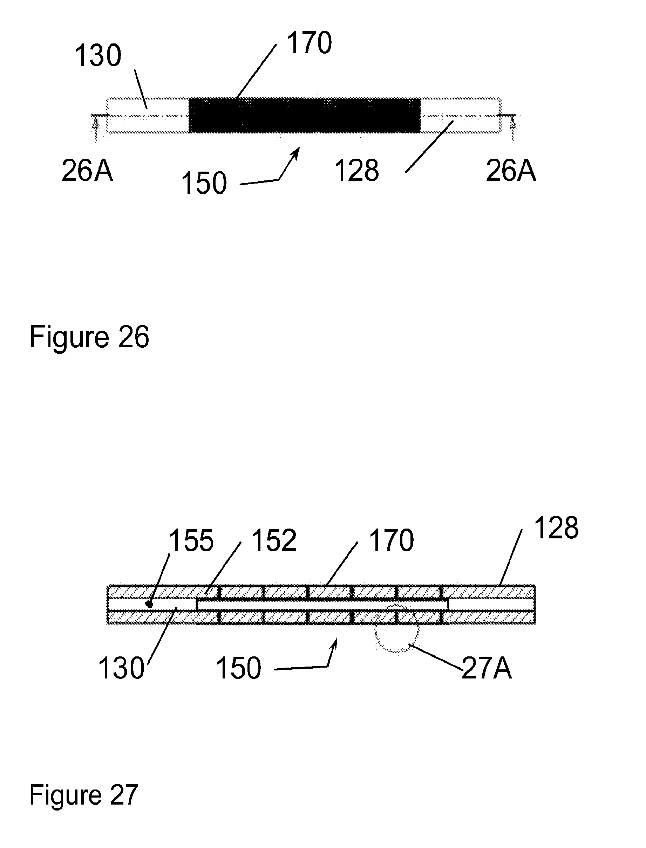

Figure 26 is an exterior view of the central with the center portion encapsulated with a resilient filler;
Figure 27 is a sectional illustration though the longitudinal axis 213A of the central segment in Figure 26 showing the filled slot with a resilient filler encapsulating the entire segment but not filling the central core; and

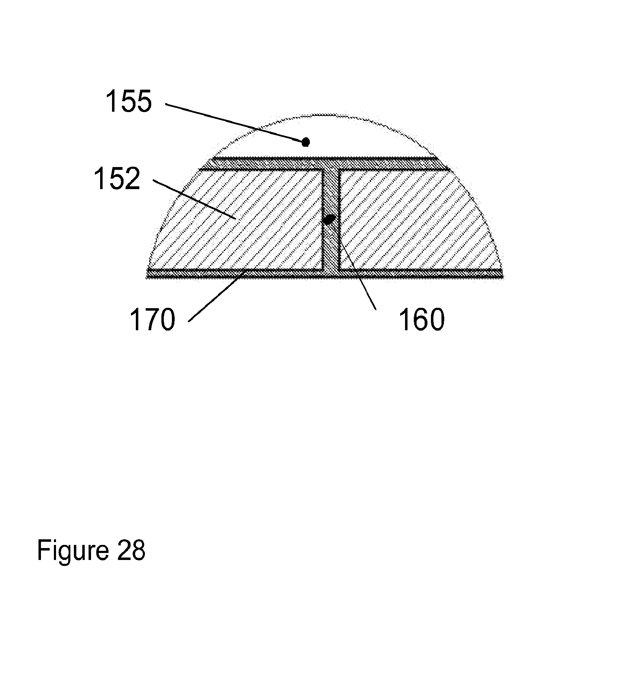

Figure 28 is a magnified view of the area 214A in Figure 27 in accordance with the invention.

### DESCRIPTION OF THE PREFERRED EMBODIMENT

For the purposes herein the terms "slit" and "slot" are used interchangeably, consistent with their definitions, as follows: slot n. 1. A narrow opening; a groove or slit: a slot for coins in a vending machine; a mail slot. A gap between a main and an auxiliary airfoil to provide space for airflow and facilitate the smooth passage of air over the wing.

For the purposes herein the term pitch as used herein is defined as: pitch - n.1 . The distance traveled by a machine screw in one revolution. 2. The distance between two corresponding points on adjacent screw threads or gear teeth. (American Heritage Dictionary, 3rd Edition, Copyright 1994)

For the purposes herein the term "cycle" shall refer to: Cycle - 1. An interval of time during which a characteristic, often regularly repeated event or sequence of events occurs: Sunspots increase and decrease in intensity in an 1 1 -year cycle. 2. a. A single complete execution of a periodically repeated phenomenon: A year constitutes a cycle of the seasons. 2b. A periodically repeated sequence of events: cycle includes two halves of the sine-wave like undulation of the slot path. (American Heritage Dictionary, 3rd Edition, Copyright 1994)

For the purposes herein the term "amplitude" shall refer to the maximum absolute value of the periodically varying quantity of the slot.

For the purposes herein the term "serpentine" shall refer to: 3 a : winding or turning one way and another <a serpentine road> b : having a compound curve whose central curve is convex. (Merham-Webster online dictionary)

For the purposes herein the term "helical", "helix" and "spiral" and interchangeable and shall refer to: 1 a : winding around a center or pole and gradually receding from or approaching it <the spiral curve of a watch sphng> b : helical c : spiral-bound <a spiral notebook> 2 : of or relating to the advancement to higher levels through a series of cyclical movements. (Merriam-Webster online dictionary)

For the purposes herein the term "frequency" shall refer to the number of times a specified phenomenon occurs within a specified interval: Frequency. 1 a. Number of repetitions of a complete sequence of values of a periodic function per unit variation of an independent variable. 1 b. Number of complete cycles of a periodic process occurring per unit time. 1 c. Number of repetitions per unit time of a complete waveform, as of an electric current. The number of times the cycles form a repetitive pattern in one unit of length is the frequency of the slot pattern. The number of cycles "C" of the slot undulations superimposed upon the circumferential path which are present in one revolution around the shaft, is referred to as the cycles per revolution. (American Heritage Dictionary, 3rd Edition, Copyright 1994)

While the present invention will be described more fully hereinafter with reference to the accompanying drawings, in which particular embodiments and methods of implantation are shown, it is to be understood at the outset that persons skilled in the art can modify the invention herein described while achieving the functions and results of this invention.

Flexible screws are useable in many other applications to join curved members together, to join misaligned holes, to absorb vibration between two components and numerous other applications. Although usable in many forms of bone connection, the bone screws disclosed are particularly useful in the intramedullary fixation of fractured or severed bone fragments.

Accordingly, the descriptions that follow are to be understood as illustrative and exemplary of specific structures, aspects and features within the broad scope of the present invention and not as limiting of such broad scope.

The invention in one embodiment relates to a flexible compression screw having one or more flexible segments within an unthreaded central section of the screw. The flexible screw can also contain a segment, or segments, that also include threads. The flexibility is created through the use of at least one helical slot formed in the center segment of the element. In other embodiments, additional flexible segments also have at least one helical slot in either the same helical rotation and pattern or in an opposite rotation and/or different pattern. In another embodiment the flexible section has a flexible segment that has at least one helical, serpentine slot within a section of the screw element that is embedded within a polymer or other flexible material so as to fill the slot with the flexible material as disclosed in US Patents 6,053,922 and 6,447,518. In an additional embodiment the flexible screw uses a hollow flexible element that encompasses a polymer or other flexible material within its central core without extending into the helical slot(s). A further embodiment uses a flexible slotted segment within the element that contains a polymer or other flexible material within the central core with the flexible material extending radially outward through the helical, serpentine slot(s). The flexible screw can further incorporate a flexible slotted segment that contains a polymer or other flexible material within the central core of the flexible segment that extends radially outward through the slot and encompasses the outer surface of the element and/or the flexible segment.

The flexible screw can have a hollow body, including leading and trailing edge, or can have a partially hollow body. The screws manufactured with a partially hollow body would have solid leading and trailing segments that are welded onto the hollow flexible segment. In some applications the entire flexible screw can be solid.

The disclosed flexible compression bone screw can also be used for treatment of chronic scapholunate instability (ligament injury between scaphoid and lunate bones of the wrist). This method has a clear advantage overt the current fixation treatment using Herbert screw by providing stability and limited motion between scaphoid and lunate bones. Using the bone screw disclosed in the pending application the leading threaded section of the flexible bone screw will be anchored in lunate bone and trailing treaded section in the scaphoid bone with flexible section place between these two bones.

In applications were additional fracture stability is required after implantation of flexible compression bone screw; bone cement or other materials can be injected in the cannulation hole. The slotted flexible section of the bone screw provides flow-through mechanism for bone cement that is used for production of a bone cement jacket around the bone screw, such that bone screw will be anchored in a highly stable manner after being implanted in the bone and specially after being implanted in a bone of reduced quality.

Numerous industrial applications are applicable for use with the disclosed flexible screws. Any application where two pre-dhlled articles that require fixation are slightly out of align can benefit from the disclosed screw. The ability to construct the screw with a small diameter in the order of one to two millimeters enables it to be used for fine, detailed work, such as guns, while the ability to enlarge the screw enables it to be used for larger applications, such furniture.

Figure 1 shows the prior art Herbert screw described by Herbert in US Patent 4,175,555 having a leading end segment 13 and a trailing end segment 11 axially spaced apart by a substantially cylindrical, center segment 15.

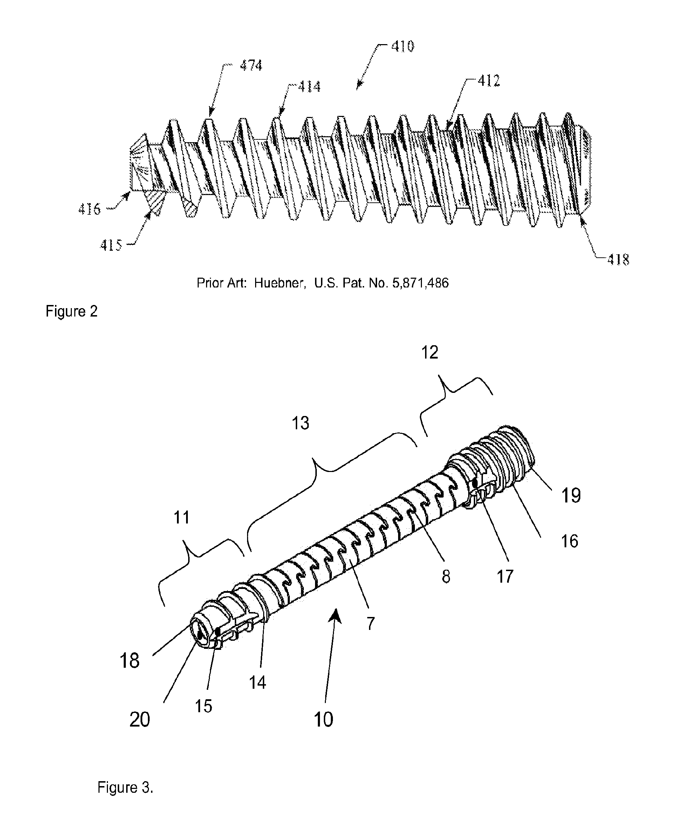

Figure 2 the Acutrak screw described by Heubner et al in US Patent 5,964768 having a tapered body with a continuous, variable pitch thread.

In Figure 3, the described flexible compression screw 10 comprises leading end segment 11 and a trailing end segment 12 axially spaced apart by a substantially cylindrical, flexible center segment 13. The leading end segment 11 is furnished with first screw thread 14 and a thread cutting recess 15 and the trailing end segment 12 has a second screw thread 16 and a thread cutting recess 17. The flexible center section 13 has a serpentine, spiral slot 8 though the shaft 7 of the center section 13 generally from the proximal end of the leading end segment 11 to the distal end of the trailing segment 12. Through the screw 10 is a hollow cavity 20 extending from the leading edge 18 to the trailing edge 19. In this, and other illustrated embodiments, the leading edge 18 is slightly beveled, however whether there is a bevel and the degree to which there is a bevel, will vary depending upon end use. The proportions between the threads would typicall be P1 >P2 or P1 <P2, although in some applications it can be beneficial for both P1 and P2 to be equal.

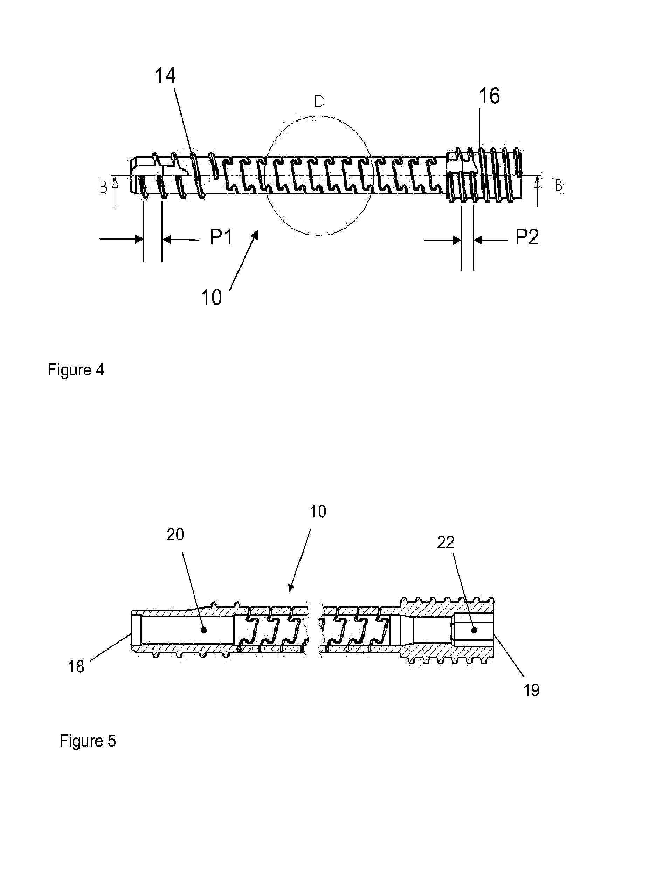

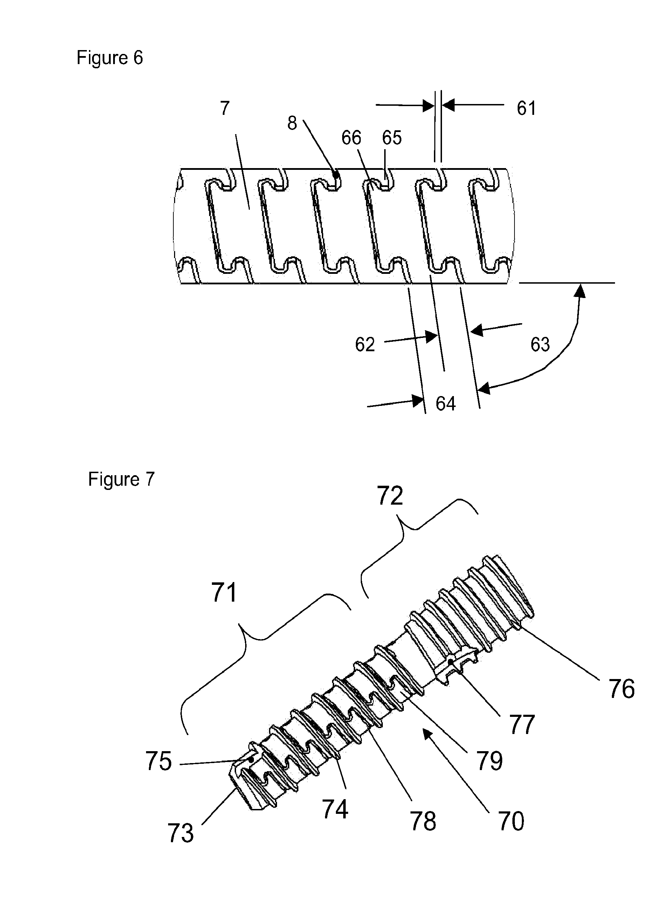

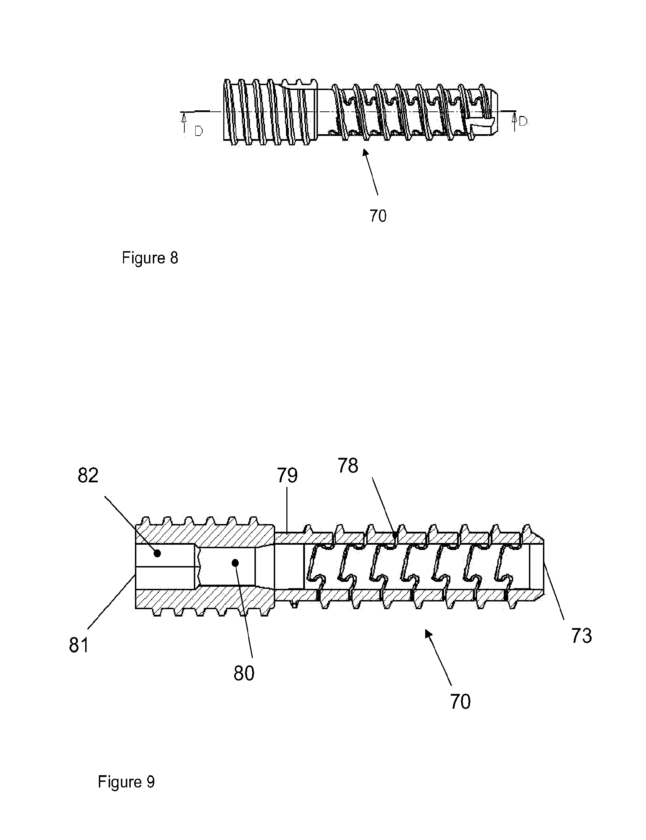

As seen in Figure 4, the threads 14 and 16, as are all threads disclosed herein, are like-handed. The screw 10 is an embodiment intended to apply compressive action; and therefore the pitch P1 of thread 14 is slightly greater than the pitch P2 of thread 16. Sections D and B-B are described in more detail in Figures 6 and 8, respectively.

Figure 5 is a sectional view of axis B-B seen in Figure 4 to illustrate the passage of the central opening 20 from the leading edge 18 extending though the screw 10 to the trailing edge 19. The trailing end 19 of the bone screw 10 is furnished with a hexagonal or similar receiving recess 22 to receive a screwdriver, or other rotational force device.

Figure 6 is an exploded view of section D in Figure 4 showing the serpentine, helical slot 8 within the shaft 7 of the central flexible section 13 of screw 10. The slot 8, having a width 61 , is cut with a general helix angle 63 of about 10 to 80 degrees with respect to the longitudinal axis of the central section 13. In this embodiment the slot 8 is cut in a serpentine pattern having an amplitude 62 and interlocking teeth 65, 66 with a pitch 64.

The slot 8 is representative of all the slots disclosed herein in that way that it is cut through the shaft 7 into the core 20. Although the slots disclosed herein are of different patterns, this is purely a function of flexibility and all have the same basic construction. In the following description of the criteria of the slots, no reference numbers specific to other figures are used, as the criteria are applicable to all slot configurations.

The helical path of the slot 8 is about 0.25 to about 5 cycles per diameter length. In order to provide the desired flexibility, while maintaining support, the width of the slot 8 should not exceed about 1.905 mm ( 0.075 of an inch) in a screw having a diameter in the range from about 2.54 to about 12.7 mm (0.10 to about 0.50 inches,) with a general width of about 0.127 to about 0.635 mm ( 0.005 to about 0.025 inches.) Or alternatively stated, the slot 8 width is between about 0.5% and about 5.0% of the diameter of the element. The helical angle ranges from about 5 degrees to about 20 degrees.

Figure 7 is an illustration of another embodiment of the flexible compression screw 70 having a leading flexible segment 71 and a trailing end segment 72. The leading flexible segment 71 is furnished with first screw thread 74 and a thread cutting recess 75. The trailing end segment 72 has a second, larger diameter screw thread 76 and a thread cutting recess 77 at the distal end of the segment 72. The leading section 71 has a serpentine, spiral slot 78 through the shaft 79 from the leading end 73 to the trailing end segment 72. Generally the pitch of the serpentine helical slot 78 will follow pitch of the helical thread 74. Alternatively, the pitch of the helical slot may be different from the pitch of the threads such that the slot cuts through the threads. Although the shaft 79 diameter can be increased, as in other embodiments, in this illustration only the minor diameter of the threads 76 is increased. In this, and subsequent embodiments, the thread 74 and slot 78 run along the flexible segment 71. This design provides stiffer flexing than in embodiments where only the slot runs in the flexing segment.

Figure 8 is a lateral elevation view of flexible compression screw 70 with section DD shown. As with the other embodiments, the second screw thread segment has a larger diameter than that of the leading, or first, screw thread segment. In this embodiment, however, the actual threads are larger while in other embodiments the threads will remain the same.

Figure 9 is the sectional view provided by section DD of Figure 8. Through the screw 70is a hollow cavity 80 extending from the leading edge 73 to the trailing edge 81. Also shown is the hexagonal or similar recess 82 to receive a screwdriver or other rotational device used in the insertion or removal of the screw 70. Also illustrated is the slot 78 cut through the wall of the shaft 79 of the screw 70, which provides the flexibility to section 71 (Figure 7) of the screw 70. The characteristics of the slot 78 are provided in Figure 6.

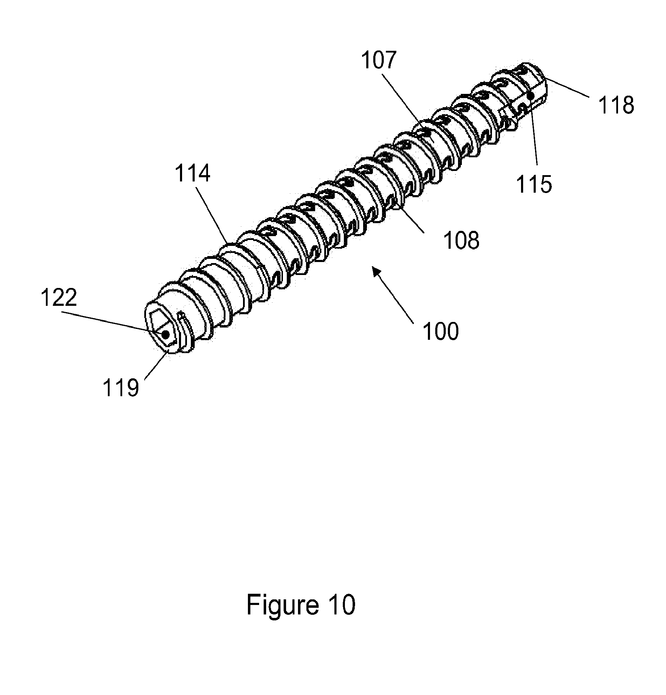

Figure 10 is an illustration of an additional embodiment of the flexible compression screw 100 having a tapered shaft 107 with the serpentine helical slot 108 extending along the shaft 107. The thread 114 is continuous from the smaller diameter leading edge 118 to the larger diameter trailing edge 119. The screw 100 has a single thread cutting recess 115. Also shown is the hexagonal or similar recess 122 to receive a screwdriver or other rotational device used in the insertion or removal of the screw 100.

Figure 11 is the lateral elevation of the tapered flexible compression screw 100 shown in Figure 10. The thread 114 extends from the leading end 118 to the trailing end 119. In practice the pitch varies continuously over the length of the screw 100 such that the leading pitch P3 is less than the trailing pitch P4 and intermediate pitches are less than proceeding thread pitch from the leading edge 118. Generally the pitch of the serpentine slot 108 will follow the pitch of the helical thread 114, but not necessarily. The serpentine slot 108 can have a pattern having about one to about 10 cycles per longitudinal revolution.

Figure 12 provides a sectional view of section CC of Figure 11. Through the screw 100 is a hollow cavity 120 extending from the leading edge 118 to the trailing edge 119. The diameter D1 of the leading edge 118 is generally less than the diameter D2 of the trailing edge 119 by an amount of about 1 millimeter or more. Also shown is the hexagonal or similar recess 122 to receive a screwdriver or other rotational device used in the insertion or removal of the screw 100. Also illustrated is the slot 108 cut through the shaft 107 of the screw 100, which provides the flexibility screw 100. The characteristics of the slot 108 are provided in Figure 6. As can be seen more clearly in Figure 12, the slot 108 extends prior to the recess 122 to prevent structural compromise.

Figure 13 illustrates another embodiment of the flexible compression screw 130 having a variable thread height from the surface of the shaft 137 over the length of the screw 130. The thread 134 which extends from the leading edge 138 has a major diameter T1 which decreases to T2 in the central section and increases to T3 at a predetermined distance prior to the trailing edge 139. Typically the T3 is greater than T1 which is greater than T2. The reduced diameter T2 in the center of the screw 130 helps to reduce the torque required to advance the screw 130. In addition, the pitch may vary over the length of the screw such that the pitch P1 of thread 134 is slightly greater than the pitch P2 of thread 134 at the trailing edge 139. As stated heretofore, the diameter of the screw, and major D1 and minor D2 diameters of the thread are largely reliant on the end use.

A variety of slot patterns are illustrated in Figure 14 A-F. The patterns are representative of patterns that can be used and are not intended to be all inclusive. As illustrated in Figure 14A, the pattern has a cycle length C, which includes a neck region NA. The wider the neck region the greater the strength of the connector, that is, the greater the torsional forces which the flexible shaft can transmit. The ability of the device to interlock is dependent in part upon the amount of overlap or dovetailing, indicated as DTA for Figure 14A and DTB for Figure 14B. The pattern of 14C, does not provide dovetailing, and requires a helix angle that is relatively small. Additional patterns, as shown in Figures 14D, 14E, 14F can have a configuration as illustrated in U.S. Patent No. 6447518.

Figure 15 illustrates another embodiment of the a flexible compression screw 50 in which the central segment 53 has two or more flexible segments 53A, 53B that can be at different helical angles 60', 60" as well as having different serpentine patterns of frequency and amplitude so as to have different flexibility. As shown in this figure, the proximal segment 53A has a stiffer, less flexible segment than the distal segment 53B due to the reduction in frequency an amplitude. An additional feature in this embodiment is the addition of a hub 59 to the distal end of the leading segment 51 with a cutting notch 55' and having a diameter approximately equal to the major diameter D1 of thread 14 which has a minor diameter D2. In turn the central segment 53 has a diameter D3 approximately equal to the major diameter D1 of the leading section thread 54 and hub 21. Reword © The threads 57 of the trailing section 52 have a minor diameter D2' approximately equal to the diameter D3 of the central section 53 and a major diameter D1 '. A cutting notch 55" is located on the proximal end of the leading edge 51.

Figure 16 illustrates another embodiment of the flexible compression screw 70 in which a flexible leading end segment 71 with helical serpentine slot 78' and a non threaded trailing end segment 72 axially spaced apart by a substantially cylindrical, flexible center segment 73 having a slot 78. The leading end segment 71 is furnished with a first screw thread 74, a thread cutting recess 75 and a helical, serpentine slot 78'.
By mixing the type of slot and/or frequency, amplitude, etc., the flexibility of the screw can be changed.

Figure 17 is an exploded view of section A in Figure 16. In this instance, the helical serpentine slot 78" is formed on the minor diameter D2 of the threaded segment 71. The slot can extend partially up the side of the thread 74 or up and over the thread 74.

Figure 18 illustrates another embodiment of the flexible compression screw 80 in which a leading threaded end segment 84, a threaded trailing end segment 82 having threads 87, are axially spaced apart by a substantially cylindrical, flexible center segment 83 with one or more concentric slots 88. The concentric slot 88 has an amplitude A and spacing C from the previous slot. The leading end segment 84 is furnished with screw thread 84 and a thread cutting recess 85 Between the leading end segment 84 and the center segment 83 is a collar 89 from which secondary cutting recess 85' is cut.

In Figure 19, the central segment 83 of Figure 18 of the present invention generally consists of a hollow tube having an outer surface 154 and a hollow central core 151 as illustrated in Figure 21. A slots 160, 160', 160", ... 160ⁿ are cut through the wall 152, shown in Figure 21 , of section of the central segment 83 to provide flexibility. Multiple circumferential slots 160', 160" ...160ⁿ are situated continually at prescribed or varying intervals over all or most of the length of the segment 83 enabling the majority of the element 83 to flex. The number of slots "n" can vary dependent upon the flexibility desired. The flexibility will be dependent upon the spacing "C" as well as the amplitude "A" of the serpentine slot 160 and the solid section 154 between slots 160. In this embodiment the slots 160 ... 160ⁿ allow for flexibility only within the flexible segment. The sections 130 and 128 of the central segment 83 that are not slotted remain relatively rigid and are used for attachment with the leading and trailing segments.

In the embodiment illustrated in Figures 20, 21 , and 22, the serpentine pattern of slot 160ⁿ⁺¹ is offset or staggered a rotational distance OFS from the adjacent slot 160ⁿ. By staggering the serpentine pattern as illustrated, the bending characteristics, i.e. the bending strength and flexibility, can be changed to provide differences or uniformity with respect to the rotational axis.

The sectional view 20A-20A of central segment 83 of Figure 18 is shown in Figure 21. A magnified view 21 B of the slot 160ⁿ is illustrated in Figure 22. The slot 160ⁿ is representative of all the slots disclosed herein in that way that it is cut through the wall 152 into the core 151. Although the slots disclosed herein are of different patterns, this is purely a function of flexibility and all have the same basic construction. The criticality to the disclosed invention lies in the ratios and dimensions rather than the process of placing a rod or tube. In the following description of the criteria of the slots, no reference numbers specific to other figures are used, as the criteria are applicable to all slot configurations.

In the embodiment illustrated in Figures 23, 24 and 25, a biocompatible resilient flexible or elastomeric material 170 fills only the slot 160 of the central segment. The exterior surface 154 of the central segment remains uncovered by the material 170 as does the interior surface 153. The addition of the elastomeric material 170 to the slot 160 provides resistance to the flexibility of the segment 83 as well as preventing tissue and scar ingrowth into the slot. It should also be noted that the elastomeric material does not necessarily have to fill all slots in the rod, with the placement of filled and unfilled slots affecting the flexibility.

In Figures 26, 27 and 28 the elastomeric material 170 encapsulates the central segment 83 as well as filling the slots 160. In this embodiment, the interior surface 230 and exterior surface 128 are covered with the elastomeric material 170 and the slots 160 are filled to prevent tissue ingrowth into the slots 160 and increase the stiffness of the spinal element. The core 155, of the encapsulated segment 150, however, remains hollow as seen in section 26A-26A in Figure 27. Although in these figures the elastomeric material 170 also fills the slots 160 passing through wall 152 as shown in Figure 28 of the enlarged section 27A, it should be noted that the elastomeric material 170 can alternatively only encapsulate the segment without filling the slots 160. Additionally, just the interior or exterior of the segment can be covered with the elastomeric material with the slots being either filled or unfilled. The encapsulation can be only at the portion of the screw that is flexible or can extend the entire length of the rod. As noted above, the addition of the elastomeric material 170 increases the resistance to flexing and is not reflective of the advantages of encapsulating segment 150 with the elastomeric material 170.

As can be practiced, any of the segments of the flexible screw can be either non-flexible or can be made flexible by the incorporation of a helical or concentric slot within the segment.

### Broad Scope of the Invention

While illustrative embodiments of the invention have been described herein, the present invention is not limited to the various preferred embodiments described herein, but includes any and all embodiments having equivalent elements, modifications, omissions, combinations (e.g., of aspects across various embodiments), adaptations and/or alterations as would be appreciated by those in the art based on the present disclosure. The limitations in the claims (e.g., including that to be later added) are to be interpreted broadly based on the language employed in the claims and not limited to examples described in the present specification or during the prosecution of the application, which examples are to be construed as non-exclusive. For example, in the present disclosure, the term "preferably" is non-exclusive and means "preferably, but not limited to." In this disclosure and during the prosecution of this application, means-plus- function or step-plus-function limitations will only be employed where for a specific claim limitation all of the following conditions are present in that limitation: a) "means for" or "step for" is expressly recited; b) a corresponding function is expressly recited; and c) structure, material or acts that support that structure are not recited. In this disclosure and during the prosecution of this application, the terminology "present invention" or "invention" may be used as a reference to one or more aspect within the present disclosure. The language of the present invention or inventions should not be improperly interpreted as an identification of chticality, should not be improperly interpreted as applying across all aspects or embodiments (i.e., it should be understood that the present invention has a number of aspects and embodiments), and should not be improperly interpreted as limiting the scope of the application or claims. In this disclosure and during the prosecution of this application, the terminology "embodiment" can be used to describe any aspect, feature, process or step, any combination thereof, and/or any portion thereof, etc. In some examples, various embodiments may include overlapping features. In this disclosure, the following abbreviated terminology may be employed: "e.g." which means "for example."

While in the foregoing we have disclosed embodiments of the invention in considerable detail, it will understood by those skilled in the art that many of these details may be varied without departing from the scope of the invention.

## Claims

1. A flexible screw (10;50;70;100;130) to connect and compress two components adjacent one another, said flexible screw being manufactured from a rigid material and having a body, said body having a diameter and a length, and multiple segments, at least one of said multiple segments having at least one helical slot (8;78;108) therethrough to form a flexible segment, at least one of said multiple segments having exterior threads (14,16;74,76;114,134) and at least one of said multiple segments being a trailing segment (12;72) having a receiving area to receive a rotational force device, **characterized in that** said at least one helical slot (8;78;108) has a serpentine pattern along the helical path to form interlocking teeth (65,66).

2. The flexible screw as claimed in claim 1, **characterized in that** one of said multiple segments is a leading end segment with an edge (18;118) and another of said multiple segments is said trailing end segment with an edge (19;81;119;139) at least one of said leading edge and said trailing edge having at least one cutting recess (15;75,77;115).

3. The flexible screw as claimed in claim 1 or 2, **characterized in that** at least one of said multiple segments of said body is hollow, and the flexible screw further comprises an elastomeric material, an application of said elastomeric material being selected from at least one of the group comprising filling at least a portion of said hollow body, filling at least a portion of said at least one slot, encompassing at least a portion of said body.

4. The flexible screw as claimed in claim 1, 2 or 3, **characterized in that** one of said multiple segments is a leading segment (11 ;71) having threads, said threads having a different pitch (64) and amplitude (62) than said threads of said trailing segment.

5. The flexible screw as claimed in anyone of the preceding claims, **characterized in that** a second of said at least one helical slot has a different pattern than, and spaced from, a first of said at least one helical slot.

6. The flexible screw as claimed in anyone of the preceding claims, **characterized in that** space between said first of said at least one helical slot and said second of said at least one helical slot (8;78;108) is inflexible.

7. The flexible screw as claimed in anyone of the preceding claims, **characterized in that** said body has a constant taper from a first of said multiple segments to said trailing edge.

8. The flexible screw as claimed in anyone of the preceding claims, **characterized in that** each of same multiple segments has a different diameter from adjoining segments.

9. The flexible screw as claimed in anyone of the preceding claims, **characterized in that**
a. a first of said multiple segments is a leading segment, having said exterior threads (14,16;74,76;114;134),
b. a second of said multiple segments is a center segment (13;53), having said helical slot (8;78;108),
c. a third of said multiple segments is said trailing segment.

10. The flexible screw as claimed in anyone of the preceding claims 1 to 9, **characterized in that**
a. a first of said multiple segments is a leading segment having said helical slot and said exterior threads (14,16;74,76;114;134),
b. a second of said multiple segments is a center segment having said helical slot (8;78;108),
c. a third of said multiple segments is a trailing segment, having said exterior threads.

11. The flexible screw as claimed in anyone of the preceding claims 1 to 9, **characterized in that**
a. a first of said multiple segments is a leading segment having said exterior threads (14,16;74,76;114;134),
b. a second of said multiple segments is a center segment having said helical slot (8;78;108) and said exterior threads,
c. a third of said multiple segments is a trailing segment (12;72), having said exterior threads.

12. The flexible screw as claimed in anyone of the preceding claims 1 to 9, **characterized in that**
a. a first of said multiple segments is a leading segment having said exterior threads (14,16;74,76;114;134) and said helical slot (8;78;108), said exterior threads having a first diameter,
b. a second of said multiple segments is a center segment, having said helical slot and said exterior threads, said exterior threads having a second diameter,
c. a third of said multiple segments is a trailing segment (12;72), having said exterior threads (14,16;74,76;114;134), said exterior threads having a third diameter.

13. The flexible screw as claimed in anyone of the preceding claims 1 to 9, **characterized in that**
a. a first of said multiple segments is a leading segment (11;71), said leading segment having exterior threads and a helical slot (8;78;108),
b. a second of said multiple segments is a center segment, said center segment having said helical slot (8;78;108),
c. a third of said multiple segments is a trailing segment (12;72).

14. The flexible screw as claimed in anyone of the preceding claims 1 to 9, **characterized in that**
a. a first of said multiple segments is a leading segment (11;71), said leading segment having exterior threads (14,16;74,76;114;134) and a helical slot (8;78;108),
b. a second of said multiple segments is a center segment,
c. a third of said multiple segments is a trailing segment (12;72).

## Patentansprüche

1. Biegsame Schraube (10, 50, 70, 100, 130), mit der zwei nebeneinander befindliche Bauteile verbunden und zusammengedrückt werden können, wobei die biegsame Schraube aus einem steifen Material gefertigt ist und einen Körper, der einen Durchmesser und eine Länge hat, sowie mehrere Segmente besitzt, wobei in mindestens einem der Segmente mindestens ein schraubenförmiger Schlitz (8; 78; 108) vorhanden ist, wodurch ein biegsames Segment gebildet wird, wobei mindestens eines der Segmente mit Außengewinden (14, 16; 74, 76; 114, 134) versehen ist und mindestens eines der Segmente ein hinteres Segment (12; 72) bildet, das mit einem Aufnahmebereich für eine Drehkraftvorrichtung versehen ist, **dadurch gekennzeichnet, dass** der mindestens eine schraubenförmige Schlitz (8; 78; 108) entlang der schraubenförmigen Bahn einen schlangenlinienförmigen Verlauf zur Bildung formschlüssiger Zinken (65, 66) besitzt.

2. Biegsame Schraube nach Anspruch 1, **dadurch gekennzeichnet, dass** eines der Segmente ein vorderes Endsegment mit einer Kante (18; 118) und ein anderes der Segmente das erwähnte hintere Segment mit einer Kante (19; 81; 119; 139) bildet, wobei mindestens eine der vorderen und der hinteren Kanten mindestens eine Schneidnute (15; 75, 77; 115) aufweist.

3. Biegsame Schraube nach einem der Ansprüche 1 oder 2, **dadurch gekennzeichnet, dass** mindestens eines der Segmente des Körpers hohl ist und die biegsame Schraube ferner ein Elastomer umfasst, wobei eine Anwendung des Elastomers aus mindestens einer Anwendung der Gruppe gewählt wurde, die das Füllen von zumindest einem Teil des genannten Hohlkörpers, das Füllen von zumindest einem Teil des mindestens einen Schlitzes oder das Umschließen von zumindest einem Teil des Körpers umfasst.

4. Biegsame Schraube nach einem der Ansprüche 1, 2 oder 3, **dadurch gekennzeichnet, dass** es sich bei einem der Segmente um ein vorderes Segment (11; 71) mit Gewinden handelt, wobei die Gewinde einen anderen Steigungswinkel (64) und eine andere Amplitude (62) besitzen als die Gewinde des hinteren Segments.

5. Biegsame Schraube nach einem der vorangegangenen Ansprüche, **dadurch gekennzeichnet, dass** ein zweiter des mindestens einen schraubenförmigen Schlitzes einen anderen Verlauf aufweist als ein erster des mindestens einen schraubenförmigen Schlitzes und im Abstand zu diesem angeordnet ist.

6. Biegsame Schraube nach einem der vorangegangenen Ansprüche, **dadurch gekennzeichnet, dass** der Bereich zwischen dem ersten des mindestens einen schraubenförmigen Schlitzes und dem zweiten des mindestens einen schraubenförmigen Schlitzes (8, 78, 108) unbiegsam ist.

7. Biegsame Schraube nach einem der vorangegangenen Ansprüche, **dadurch gekennzeichnet, dass** der Körper eine konstante Verjüngung zwischen einem ersten der Segmente und der hinteren Kante aufweist.

8. Biegsame Schraube nach einem der vorangegangenen Ansprüche, **dadurch gekennzeichnet, dass** jedes der Segmente einen anderen Durchmesser besitzt als die benachbarten Segmente.

9. Biegsame Schraube nach einem der vorangegangenen Ansprüche, **dadurch gekennzeichnet, dass**:
a. ein erstes der Segmente ein vorderes Segment bildet, das mit den erwähnten Außengewinden (14, 16, 74, 76, 114, 134) versehen ist,
b. ein zweites der Segmente ein mittleres Segment (13, 53) bildet, das mit dem erwähnten schraubenförmigen Schlitz (8, 78, 108) versehen ist,
c. ein drittes der Segmente ein hinteres Segment bildet.

10. Biegsame Schraube nach einem der vorangegangenen Ansprüche 1 bis 9, **dadurch gekennzeichnet, dass**:
a. ein erstes der erwähnten Segmente ein vorderes Segment bildet, das mit dem schraubenförmigen Schlitz und den erwähnten Außengewinden (14, 16, 74, 76, 114, 134) versehen ist,
b. ein zweites der Segmente ein mittleres Segment bildet, das mit dem schraubenförmigen Schlitz (8, 78, 108) versehen ist,
c. ein drittes der erwähnten Segmente ein hinteres Segment bildet, das mit den erwähnten Außengewinden versehen ist.

11. Biegsame Schraube nach einem der vorangegangenen Ansprüche 1 bis 9, **dadurch gekennzeichnet, dass**:
a. ein erstes der Segmente ein vorderes Segment bildet, das mit den erwähnten Außengewinden (14, 16, 74, 76, 114, 134) versehen ist,
b. ein zweites der Segmente ein mittleres Segment bildet, das mit dem schraubenförmigen Schlitz (8, 78, 108) und den erwähnten Außengewinden versehen ist,
c. ein drittes der erwähnten Segmente ein hinteres Segment (12, 72) bildet, das mit den erwähnten Außengewinden versehen ist.

12. Biegsame Schraube nach einem der vorangegangenen Ansprüche 1 bis 9, **dadurch gekennzeichnet, dass**:
a. ein erstes der erwähnten Segmente ein vorderes Segment bildet, das mit den erwähnten Außengewinden (14, 16, 74, 76, 114, 134) und dem schraubenförmigen Schlitz (8, 78,108) versehen ist, wobei die Außengewinde einen ersten Durchmesser aufweisen,
b. ein zweites der Segmente ein mittleres Segment bildet, das mit dem schraubenförmigen Schlitz und mit den Außengewinden versehen ist, wobei die Außengewinde einen zweiten Durchmesser aufweisen,
c. ein drittes der erwähnten Segmente ein hinteres Segment (12, 72) bildet, das mit den erwähnten Außengewinden (14, 16, 74, 76, 114, 134) versehen ist, wobei die Außengewinde einen dritten Durchmesser aufweisen.

13. Biegsame Schraube nach einem der vorangegangenen Ansprüche 1 bis 9, **dadurch gekennzeichnet, dass**:
a. ein erstes der erwähnten Segmente ein vorderes Segment (11, 71) bildet, wobei dieses vordere Segment mit Außengewinden und einem schraubenförmigen Schlitz (8, 78,108) versehen ist,
b. ein zweites der Segmente ein mittleres Segment bildet, wobei dieses mittlere Segment mit dem erwähnten schraubenförmigen Schlitz (8, 78,108) versehen ist,
c. ein drittes der erwähnten Segmente ein hinteres Segment (12, 72) bildet.

14. Biegsame Schraube nach einem der vorangegangenen Ansprüche 1 bis 9, **dadurch gekennzeichnet, dass**:
a. ein erstes der erwähnten Segmente ein vorderes Segment (11, 71) bildet, wobei dieses vordere Segment mit einem Außengewinde (14, 16, 74, 76, 114, 134) und einem schraubenförmigen Schlitz (8, 78,108) versehen ist,
b. ein zweites der Segmente ein mittleres Segment bildet,
c. ein drittes der erwähnten Segmente ein hinteres Segment (12, 72) bildet.

## Revendications

1. Vis flexible (10, 50, 70, 100, 130) pour le raccordement et la compression de deux composants adjacents, laquelle vis est en matériau rigide et a un corps avec un diamètre et une longueur ainsi que différents segments, dont au moins un des segments présente au moins une rainure hélicoïdale (8; 78; 108) pour former ainsi un segment flexible, dont au moins un des segments est équipé de filetages extérieurs (14,16; 74,76; 114,134) et au moins un des segments forme un segment arrière (12; 72) qui est équipé d'une zone de réception pour recevoir un dispositif de force rotative, **caractérisée en ce que** la rainure hélicoïdale (8; 78; 108) dont il y en a au moins une, serpente le long de la voie hélicoïdale pour former ainsi des dents engrenés (65, 66).

2. Vis flexible selon la revendication 1, **caractérisée en ce qu'**un des segments est un segment de bout avant avec un bord (18; 118) et un autre des segments est ledit segment arrière avec un bord (19; 81; 119; 139) dont au moins un des bords avants et le bord arrière a au moins un évidement découpe (15; 75,77; 115).

3. Vis flexible selon la revendication 1 ou 2, **caractérisée en ce qu'**au moins un des segments du corps est creux, et la vis flexible comporte en outre un matériau élastomère, l'application du matériau élastomère étant choisi pour au moins un du groupe, pour remplir au moins une partie dudit corps creux, remplir au moins une partie de la rainure, dont il y en a au moins une, enrober au moins une partie du corps.

4. Vis flexible selon la revendication 1, 2 ou 3, **caractérisée en ce qu'**un des segments est un segment avant (11; 71) avec filetages, qui ont un autre pas (64) et une autre amplitude (62) que les filetages du segment arrière.

5. Vis flexible selon l'une des revendications précédentes, **caractérisée en ce qu'**une deuxième rainure hélicoïdale, dont il y en a au moins une, a un autre modèle et est écartée par rapport à une première rainure hélicoïdale, dont il y en a au moins une.

6. Vis flexible selon l'une des revendications précédentes, **caractérisée en ce que** l'espace entre la première rainure hélicoïdale, dont il y en a au moins une et la deuxième rainure hélicoïdale dont il y en a au moins une (8, 78, 108) est non-flexible.

7. Vis flexible selon l'une des revendications précédentes, **caractérisée en ce que** le corps a une forme conique permanente d'un premier des segments jusqu'au bord arrière.

8. Vis flexible selon l'une des revendications précédentes, **caractérisée en ce que** chacun des segments a un diamètre différent par rapport aux segments adjacents.

9. Vis flexible selon l'une des revendications précédentes, **caractérisée en ce que**
a. un premier des segments est un segment avant équipé desdits filetages extérieurs (14,16, 74,76, 114, 134),
b. un deuxième des segments est un segment central (13, 53) équipé de ladite rainure hélicoïdale (8, 78, 108),
c. un troisième des segments est le segment arrière.

10. Vis flexible selon l'une des revendications précédentes 1 à 9, **caractérisée en ce que**
a. un premier desdits segments est un segment avant équipé de la rainure hélicoïdale et desdits filetages extérieurs (14,16, 74,76, 114, 134),
b. un deuxième des segments est un segment central équipé de la rainure hélicoïdale (8, 78, 108),
c. un troisième desdits segments est un segment arrière équipé desdits filetages extérieurs.

11. Vis flexible selon l'une des revendications précédentes 1 à 9, **caractérisée en ce que**
a. un premier des segments est un segment avant équipé desdits filetages extérieurs (14,16, 74,76, 114, 134),
b. un deuxième des segments est un segment central équipé de la rainure hélicoïdale (8, 78, 108) et desdits filetages extérieurs,
c. un troisième desdits segments est un segment arrière (12, 72) équipé desdits filetages extérieurs.

12. Vis flexible selon l'une des revendications précédentes 1 à 9, **caractérisée en ce que**
a. un premier des segments est un segment avant équipé desdits filetages extérieurs (14,16, 74,76, 114, 134) et de ladite rainure hélicoïdale (8, 78, 108), les filetages extérieurs ayant un premier diamètre,
b. un deuxième desdits segments est un segment central équipé de la rainure hélicoïdale et des filetages extérieurs, les filetages extérieurs ayant un deuxième diamètre,
c. un troisième desdits segments est un segment arrière (12, 72) équipé des filetages extérieurs (14,16; 74,76; 114; 134), les filetages extérieurs ayant un troisième diamètre.

13. Vis flexible selon l'une des revendications précédentes 1 à 9, **caractérisée en ce que**
a. un premier desdits segments est un segment avant (11, 71), équipé de filetages extérieurs et d'une rainure hélicoïdale (8, 78, 108),
b. un deuxième des segments est un segment central, équipé de ladite rainure hélicoïdale (8, 78, 108),
c. un troisième desdits segments est un segment arrière (12, 72).

14. Vis flexible selon l'une des revendications précédentes 1 à 9, **caractérisée en ce que**
a. un premier desdits segments est un segment avant (11, 71) équipé de filetage extérieur (14,16, 74,76, 114, 134) et d'une rainure hélicoïdale (8, 78, 108),
b. un deuxième desdits segments est un segment central,
c. un troisième desdits segments est un segment arrière (12, 72).
